# EUROPEAN PATENT SPECIFICATION

(11) **EP 0 779 893 B1**
(45) Date of publication and mention of the grant of the patent: **27.06.2001**
(21) Application number: 95934409.4
(22) Date of filing: 07.09.1995
(51) Int. Cl.: C07H 21/00

(54) **OLIGONUCLEOTIDE PRODRUGS**
PRODRUG-OLIGONUKLEOTIDE
PROMEDICAMENTS OLIGONUCLEOTIDIQUES

(30) Priority: 07.09.1994 US 302132
(43) Date of publication of application: 25.06.1997
(73) Proprietor: HYBRIDON, INC., Cambridge, Massachusetts 02139 (US)
(72) Inventor: IYER, Radhakrishnan, P., Shrewsbury, MA 01545 (US); YU, Dong, Somerville, MA 02144 (US); AGRAWAL, Sudhir, Shrewsbury, MA 01545 (US); DEVLIN, Theresa, Jamaica Plain, MA 02130 (US)
(74) Representative: Vossius, Volker, Dr.
(86) International application number: US9511477
(87) International publication number: WO9607392

(56) References cited:
- WO-A-94/26764
- US-A- 4 547 569
- US-A- 4 958 013
- BIOORGANIC CHEMISTRY, vol. 23, 1995 pages 1-21, R.P. IYER ET AL. 'Prodrugs of Oligonucleotides'
- BIOORGANIC MED. CHEM. LETT., vol. 4, no. 20, 1994 pages 2471-6, R.P. IYER ET AL. 'Stereospecific Bio-Reversibility of Dinucleotide S-Alkyl Phosphorothiolates to Dinucleoside Phosphorothioates'
- SYNTH. COMMUN., vol. 25, no. 18, 1995 pages 2739-49, R.P. IYER AT AL. 'Synthesis of Iodoalkylacylates and Their Use in the Preparation of S-Alkyl Phosphorothiolates'
- BIOORGANIC MED. CHEM. LETT., vol. 5, no. 6, 1995 pages 563-8, J.L. IMBACH ET AL. 'The Prooligonucleotide Approach'
- J. MED. CHEM., vol. 37, no. 8, 1994 pages 1857-64, J.E. STARRETT ET AL. 'Synthesis, Oral Bioavailability Determination and in Vitro Evaluation of Prodrugs of the Antiviral Agent 9-(2-(Phosphonomethoxy)ethyl)adenine (PMEA)' cited in the application
- NUCLEOSIDES, NUCLEOTIDES, vol. 10, 1991 pages 303-6, H. SELIGER ET AL. 'Specific Interchain Introduction of Reporter Groups into Oligonucleotides as Substituents at Internucleotidic Linkages'

### SEQUENCE LISTING

(1) GENERAL INFORMATION:
   (i) APPLICANT: Hybridon, Inc.
   (ii) TITLE OF INVENTION: Oligonucleotide Prodrugs
   (iii) NUMBER OF SEQUENCES: 10
   (iv) CORRESPONDENCE ADDRESS:
      (A) ADDRESSEE: Lappin & Kusmer
      (B) STREET: 200 State Street
      (C) CITY: Boston
      (D) STATE: Massachusetts
      (E) COUNTRY: USA
      (F) ZIP: 02109
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Kerner, Ann-Louise
      (B) REGISTRATION NUMBER: 33,523
      (C) REFERENCE/DOCKET NUMBER: HYZ-025PCT
   (ix) TELECOMMUNICATION INFORMATION:
      (A) TELEPHONE: 617-330-1300
      (B) TELEFAX: 617-330-1311
(2) INFORMATION FOR SEQ ID NO:1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:1:
   CTCTCGCACC CATCTCTCTC CTTCT 25
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA/RNA
   (iii) HYPOTHETICAL: NO
   (iv) ANTI-SENSE: YES
   (xi) SEQUENCE DESCRIPTION: SEQ ID NO:2:
   CUCUCGCACC CATCTCTCTC CUUCU 25
(2) INFORMATION FOR SEQ ID NO:3:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:3:
   AAATGT 6
(2) INFORMATION FOR SEQ ID NO:4:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:4:
   CGGCAA 6
(2) INFORMATION FOR SEQ ID NO:5:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (xi) SEQUENCE DESCRIPTION: SEQ ID NO:5:
   UGCCAG 6
(2) INFORMATION FOR SEQ ID NO:6:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:6:
   GTAAAACGAC GGCCAGT 17
(2) INFORMATION FOR SEQ ID NO:7
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:7:
   GTATTCAAAG GAGTACC 17
(2) INFORMATION FOR SEQ ID NO:8:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (xi) SEQUENCE DESCRIPTION: SEQ ID NO:8:
   GAGCAUCACG GUGAGCG                                                17
(2) INFORMATION FOR SEQ ID NO:9
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA/mRNA
   (iii) HYPOTHETICAL: NO
   (iv) ANTI-SENSE: YES
   (xi) SEQUENCE DESCRIPTION: SEQ ID NO:9:
   GAATGACTGA TTGAGTGACT GAATGCCCGT                                  30
(2) INFORMATION FOR SEQ ID NO:10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA/mRNA
   (iii) HYPOTHETICAL: NO
   (iv) ANTI-SENSE: YES
   (xi) SEQUENCE DESCRIPTION: SEQ ID NO:10:
   CAGUGACUGA CUGAGCGACU GAACUCCCGT                                  30

## Description

### BACKGROUND OF THE INVENTION

This invention relates to antisense therapy. More particularly, this invention relates to compositions and methods for enhancing the cellular uptake of antisense oligonucleotides.

New- chemotherapeutic agents have been developed which are capable of modulating cellular and foreign gene expression. These agents, called antisense oligonucleotides, are single-stranded oligonucleotides which bind to a target nucleic acid molecule according to the Watson-Crick or Hcogsteen rule of base pairing, and in doing so, disrupt the function of the target by one of several mechanisms: by preventing the binding of factors required for normal transcription, splicing, or translation; by triggering the enzymatic destruction of RNA by RNase H, or by destroying the target via reactive groups attached directly to the antisense oligonucleotide. Thus, they have become widely used research tools for inhibiting gene expression sequence specifically, and are under investigation for possible use as therapeutic agents (see, e.g., Lisciewicz et al. (*Proc. Natl. Acad. Sci. (USA)* (1993) 90:380-386); Bayever et al. (1992) *Antisense Res. Development* 2:109-110).

In order for antisense molecules to have therapeutic value, they must have the ability to enter a cell and contact target endogenous nucleic acids. Furthermore, they must be able to withstand the rigors of the highly nucleolytic environment of the cell and/or body.

Recent studies have shown that oligonucleotides with certain modifications, such as artificial internucleotide linkages, not only render the oligonucleotides resistant to nucleolytic degradation (see, e.g., Agrawal et al. (1988) ***Proc. Natl. Acad. Sci. (USA)* 85:**7079-7083; Agrawal et al. (1989) ***Proc. Natl. Acad. Sci. (USA)* 86**:7790-7794; Gao et al. (1990) ***Antimicrob. Agents Chem.* 34**:808; and Storey et al. (1991) ***Nucleic Acids Res.* 19**:4109), but also may increase cellular uptake of the oligonucleotide. For example, oligonucleotides with phosphorothioate or methylphosphonate internucleotide linkages have been found to bind to, and to be taken up by, cells more readily than phosphodiester-linked oligonucleotides (Zhao et al. (1993) ***Antisense Res. Dev.* 3**:53-56).

Oligonucleotide uptake is saturable, sequence-independent, and temperature and energy dependent. While there is some evidence to suggest that such uptake may occur through a 80,000 dalton membrane protein (Loke et al. (1989) *Proc. Natl. Acad. Sci. (USA)* **86**:3474; Yakubov et al. (1989) *Proc. Natl. Acad. Sci. (USA)* **86**:6454), the gene for this protein has not yet been cloned or
characterized. One study suggests internalization of the oligonucleotide is by a caveolar, protocytotic mechanism rather than by endocytosis (Zamecnick (1994) ***Proc. Natl. Acad. Sci. (USA)* 91:**3156). Whether oligonucleotides are internalized via a receptor-mediated endocytotic pathway, a pinocytic mechanism, or a combination of both remains poorly understood.

To improve on their cellular uptake, oligonucleotides have also been modified in ways other than those described above. For example, an oligonucleotide with improved cellular uptake has been disclosed having at least one nucleotide residue covalently linked at its 2' position with various molecules including an amino acid, polypeptide, protein, sugar, sugar phosphate, neurotransmitter, hormone, cyclodextrin, starch, steroid, or vitamin (WO 93/23,570). Enhanced cellular uptake of biotinylated oligonucleotide in the presence of avidin has also been demonstrated (Pardridge et al. (1991) ***FEBS Lett.*** **288**:30-32). In addition, phosphodiester-linked oligodeoxynucleotides have been introduced into cells by the pore-forming agent streptolysin O (Barry et al. (1993) ***Biotechniques* 15:**1016-1018), and a liposomal preparation including cationic lipid has been shown to enhance the cellular uptake of antisense molecules targeted to a portion of a human intercellular adhesion molecule (Bennett et al. (1992) ***Mol. Pharmacol.* 41**:1023-1033). Phosphodiester-linked oligonucleotides bearing a 5'-cholesteryl modification show increased cellular uptake and biological effects (Krieg et al. (1993) ***Proc. Natl. Acad. Sci. (USA)* 90**:1048). In addition, antibody-targeted liposomes have been used to enhance the cellular uptake of oligonucleotides targeted to HLA class I molecules expressed by HIV-infected cells (Zelphati et al. (1993) ***Antisense Res. Dev.* 3**:323-338).

Specific non-oligonucleotidic, metabolically unstable molecules useful as medicaments have been prepared in the form of precursors or "prodrugs" which are capable of undergoing a chemical or enzyme-mediated transformation within the target organ or cell to release the therapeutic molecule (see, Sundgaard, *in Bio-reversible Carriers in Drug Design. Theory and Application* (Roche, ed.) Pergamon Press, NY (1987) pp. 13-94). For example, acyloxyalkyl ester-type groups have been appended to carboxylic groups of the β-lactam antibiotics such as pivampicillin, talampicillin, and bacampicillin to form prodrug derivatives of ampicillin (see, e.g., Daehne et al. (1970) *J*. *Med. Chem.* 13:607; Bodin et al. (1975) *Antimicrob. Agents Chemother.* 8:518; Clayton et al. (1976) *J. Med. Chem.* 19:1385). Phosphonate prodrugs of antiviral agents such as 9-[2-(phosphonomethyoxy)-ethyl]adenine (PMEA) (Starrett et al. (1994) *J. Med. Chem.* 37:1857-1864) and trisodium phosphonoformate (foscarnet sodium) (lyer et al. (1989) *Tetrahedron Lett.* 30:7141-7144) have been prepared to increase oral availability. Phosphate groups have been appended to N-phosphomethyl dipeptides to form prodrugs of zinc protease neutral endopeptidase, an antihypertensive (De Lombaert et al. (1994) *J. Med. Chem.* 37:498-511). Anticancer prodrugs of butyric acid have been prepared (Nudelman et al. (1994) *J. Med. Chem.* 35:687-694). In addition, anti-herpes prodrugs composed of diphosphate analogs of 5-iodo-2'-deoxy-uridine-5'-diphosphate have been reported (Jennings et al. (1992) *J. Chem. Soc. Perkin Trans.* I:2196-2202).

Berber, I. et al., 1995, Bioorganic Med. Chem. Lett. 5, *563-568* (published after the priority date of the present invention), port of which has been presented at the "3^{rd} Cambridge Symposium: oligonucleotides and analogues", Sept. 5-8, 1993, Cambridge, England, describe the hydrolysis of a 3',5'-dinucleosidyl phosphorothiolate triester to its corresponding phosphorothioate diester.

However, prodrugs of antisense oligonucleotides heretofore have not existed, and insufficient uptake of modified and unmodified oligonucleotides remains a problem both *in vitro* and *in vivo*. Thus, there remains a need for improved compositions and methods for enhancing the cellular uptake and metabolic stability of antisense oligonucleotides. Such enhancement would ultimately result in an increased efficacy of antisense oligonucleotides and a reduction in the dose administered that have to be used. Ideally, such compositions and methods will also be useful for increasing the general lipid solubility of oligonucleotides.

### SUMMARY OF THE INVENTION

This invention provides improved compositions and methods for enhancing the. cellular uptake and metabolic stability of antisense oligonucleotides, and for increasing the cellular and general *in vivo* lipid solubility of such oligonucleotides. Also provided are antisense oligonucleotides with enhanced cellular uptake, increased oral bioavailability, sustained or controlled release characteristics, reduced toxicity, and increased ability to cross physiological barriers.

It has been discovered that the *in vivo* half-life and uptake of antisense oligonucleotides into cells can be enhanced by the reversible derivatization of these oligonucleotides with a lipophilic chemical group. Covalent attachment of the lipophilic group to the oligonucleotides renders them less ionic and more susceptible to transport through cell membranes than their underivatized parent. Once inside the cell or body, an endogenous enzyme cleaves the lipophilic group from the derivatized oligonucleotide, thereby regenerating the parent oligonucleotide. This discovery has been exploited to produce synthetic, reversibly derivatized antisense oligonucleotides or "oligonucleotide prodrugs" and methods of their use.

In one aspect of the invention, an oligonucleotide prodrug is provided. As used herein, the term "oligonucleotide prodrug" refers to a molecule including a plurality of nucleotides that are covalently linked together, 3' to 5', 5' to 3', 3' to 3', 5' to 2', 2' to 5, 2' to 3', or 3' to 2', and which has been masked or derivatized with a chemical group that causes the oligonucleotide to become more lipophilic, and hence to pass through lipid membranes with more ease than can the parent molecule. In addition, the oligonucleotide in its "prodrug" form may be less susceptible to degradation than its parent, but like its parent, may hybridize to other nucleic acids having a complementary nucleotide sequence. When in contact with certain enzymes in the cell, tissue, or body, the prodrug is cleaved such that the parent oligonucleotide is regenerated.

The oligonucleotide prodrug includes at least six covalently linked nucleotides. At least one of these nucleotides is derivatized with a lipophilic chemical group reversibly and covalently attached to a 5' phosphorothioate or a 3' phosphorothioate of the nucleotide, or to an internucleotidic phosphorothioate linkage.

As used herein, the term "nucleotide" refers to deoxyribonucleotides and analogs thereof, including analogs having a cyclic sugar and/or modified bases and riboxynucleotides and analogs thereof. In some embodiments, the oligonucleotide prodrug is "a hybrid oligonucleotide," i.e., it includes at least one ribonucleotide or analog thereof, and at least one deoxyribonucleotide or analog thereof. In one specific embodiment, the ribonucleotide analog is a 2-O-alkyl ribonucleotide such as a 2-O-methyl.

The lipophilic group attached to the nucleotide includes an ester, and the prodrug reacts with a cellular or tissue enzyme which cleaves the lipophilic group from the derivatized nucleotide. In preferred embodiments, the enzyme is an esterase and the lipophilic group comprises an ester.

The lipophilic chemical group covalently attached to the nucleotide is an isobutyryloxymethyl group or a propanoyloxymethyl group.

In other embodiments, the oligonucleotide prodrug is "chimeric". As used herein, "chimeric" refers to an oligonucleotide composed of more than one type of nucleotide. In one particular embodiment, the oligonucleotide prodrug consists of at least two different nucleotides such as a phosphodiester, carbamate, phosphorothioate, phosphorodithioate, acetamidate, phosphoramidate, phosphodiester, alkylphosphonate, carbonate, alkylphosphonothioate, phosphoramidite, or carboxymethyl ester, or any analog that is isosteric with the base sugar and internucleoside moiety of an unmodified oligonucleotide. In other embodiments, the oligonucleotide prodrug may be branched, i.e., may comprise two oligonucleotide sequences linked together via their 3' and/or 2' ends.

The invention also provides a pharmaceutical formulation including an oligonucleotide prodrug. In some embodiments, this pharmaceutical formulation contains an oligonucleotide prodrug that is complementary to a region of a viral nucleic acid, and also contains another antiviral agent in addition to the prodrug. In one particular embodiment, the oligonucleotide prodrug in the pharmaceutical formulation is complementary to a first region of the viral nucleic acid, and the antiviral agent is an antisense oligonucleotide having a nucleotide sequence complementary to a second region of the viral nucleic acid which does not overlap with the first region. In yet another embodiment, the pharmaceutical formulation includes an orally tolerable carrier.

A method of increasing the cellular uptake and intracellular concentration of an exogenous oligonucleotide is also provided by the present invention. In this method, a cell is treated or contacted with the pharmaceutical formulation described above. Once inside the cell a cellular enzyme cleaves the lipophilic group on the prodrug from the reversibly derivatized nucleotide, thereby regenerating the parent oligonucleotide from the oligonucleotide prodrug. In this way, the intracellular concentration of the oligonucleotide is increased. In some preferred embodiments, the lipophilic group is cleavable by an esterase.

In another aspect of the invention, a method of treating a cell for viral infection, or of preventing viral infection in the cell, is provided. In this method, the cell is contacted with an oligonucleotide prodrug having a nucleotide sequence complementary to a portion of the nucleic acid of a virus. The oligonucleotide prodrug enters the cell wherein an esterase cleaves the lipophilic chemical group from the derivatized nucleotide, thereby releasing the parent oligonucleotide. The oligonucleotide then hybridizes to a complementary portion of the viral nucleic acid. Thus, the invention provides a useful composition for treating inadvertently infected cell culture lines. Contamination of cell lines with viruses or mycoplasma can be eliminated by using the compositions according to the invention.

In yet another aspect, the invention provides a method of increasing the intracellular or ***in vivo*** lipid solubility and bioavailability of an oligonucleotide. In this method an oligonucleotide is derivatized to form an oligonucleotide prodrug which is more lipid soluble and bioavailable than the oligonucleotide. As described above, the prodrug includes at least six covalently linked nucleotides, at least one of which nucleotide has a 5' phosphorothioate, a 3' phosphorothioate, or an internucleotidic phosphorothioate linkage to which is reversibly and covalently attached a lipophilic chemical group, and which is cleavable with a cellular esterase.

### BRIEF DESCRIPTION OF THE DRAWINGS

The foregoing and other aspects of the present invention, the various features thereof, as well as the invention itself may be more fully understood from the following description, when read together with the accompanying drawings in which:
FIG. 1 is a diagrammatic representation of a generalized scheme depicting the conversion of an oligonucleotide prodrug to an oligonucleotide, wherein "Z" is a functional group, "X" is O, S, or NR (R is alkyl or aryl), Y is O or S, R is alkyl, aryl, ar-alkyl, heterocyclic group, fatty acid, or steroid, " R" " is a branching group, and "Q" is a heteroatom such as O or S, or is a covalent bond;
FIG. 2 is diagrammatic representation of the action of an enzyme on various lipophilic groups of different oligonucleotide prodrugs to yield the same parent oligonucleotide;
FIG. 3 shows a diagrammatic representation of the general structure of a steroid nucleus which can be covalently attached to a nucleotide via any site on the steroid via a Z (amide or ester) group;
FIG. 4 is a schematic representation of the bioactivation of acyloxyalkyl ester-type prodrug **1** with esterases to yield oligonucleotide **5**;
FIG. 5 is a diagrammatic representation of the preparation of iodoalkylacylates **10a-d** and the treatment of Rₚ **2** or Sₚ **2** with iodoalkylacylates **10a-d** to yield the S-alkyl dinucleoside, phosphorothioates 3a-3d;
FIG. 6 is a schematic representation of the hydrolysis of d(TpsT) esters **3a-c** to yield compound **4** and parent oligonucleotide **2**;
FIG. 7A is a collection of reversed-phase HPLC profiles of the time course of hydrolysis of Rₚ **3b** with human serum, wherein the arrows indicate the retention times in minutes;
FIG. 7B is a collection of reversed phase HPLC profiles of the time course of hydrolysis of Sₚ **3b** with human serum, wherein the arrors indicate the retention times in minutes;
FIG. 8 shows the ³¹P-NMR spectra and autoradiogram of a polyacrylamide gel of a PS/PO containing parent oligonucleotide (A), the oligonucleotide prodrug (B), and the oligonucleotide prodrug after incubation with an esterase for 24 hours (C); and
FIG. 9 is an autoradiogram of parent oligonucleotide (lanes 1 and 3), prodrug oligonucleotide (lanes 2 and 4), and prodrug oligonucleotide after incubation with an esterase for 36 hours.

### DETAILED DESCRIPTION OF THE PREFERRED EMBODIMENTS

The patent and scientific literature referred to herein establishes the knowledge that is available to those with skill in the art.

In order for antisense oligonucleotides to elicit their therapeutic action as inhibitors of gene expression, they must be taken up by cells and internalized. However, if the oligonucleotide is polyionic and of high molecular weight, its ability to cross lipid membranes is reduced; oligonucleotides that carry less negative charges are known to be taken up by cells more efficiently (Temsamani et al. (1994) ***Antisense Res. Dev.* 4:**35-42).

The present invention provides a method of improving oligonucleotide uptake through lipid membranes into cells, thereby increasing the efficacy of treatment and reducing the dose of antisense oligonucleotide required. In this approach, oligonucleotide-containing prodrugs have been designed which undergo an enzyme-mediated transformation near or within the target organ, tissue, or cell to release the functional parent antisense oligonucleotide. The oligonucleotide prodrugs are oligonucleotides that have been reversibly derivatized to become lipophilic, less ionic oligonucleotide conjugates having the ability to enter cells by passive diffusion through cell membranes and also to get transported across various physiologic barriers including the blood-brain barrier.

The oligonucleotide prodrugs include at least six, and preferably 10 to 30 nucleotides. The 3' terminus of one nucleotide is covalently linked to the 5' terminus of the next nucleotide. The nucleotides may be deoxyribonucleotides or analogs thereof, ribonucleotides or analogs thereof, or a combination of deoxyribonucleotides, deoxyribonucleotide analogs, ribonucleotides, and ribonucleotide analogs, thereby forming a chimeric oligonucleotide prodrug.

The term "nucleotide analog" as used herein encompasses a nucleotide not found naturally in *vivo* and having a synthetic group attached or replacing its 3' or 5' terminal chemical groups. Thus a nucleotide analog forms an internucleotide linkage other than a phosphodiester between the 5' end of one nucleotide and the 3' end of another nucleotide in which the 5' nucleotide phosphate has been replaced with any number of chemical groups. Preferable synthetic linkages include alkylphosphonates, phosphate esters, alkylphosphonates, phosphorothioates, phosphorodithioates, carbonates, alkylphosphonothioates, phosphoramidates, carbamates, phosphate triesters, acetamidate, and carboxymethyl esters.

The term "nucleotide analog" also encompasses nucleotides with a modified base and/or sugar. For example, a 3', 5'-substituted nucleotide is a modified nucleotide having a sugar which, at both its 3' and 5' positions is attached to a chemical group other than a hydroxyl group (at its 3' position) and other than a phosphate group (at its 5' position). A modified nucleotide may also be a capped species. In addition, unoxidized or partially oxidized nucleotides having a substitution in one nonbridging oxygen per nucleotide in the molecule are also considered to be modified oligonucleotides. Also considered as modified nucleotides are those having nuclease resistance-conferring bulky substituents at their 3' and/or 5' end(s) and/or various other structural modifications not found ***in vivo*** without human intervention. Modifications may also include a substitution at the phosphate group. For example, the oxygen at the 5' phosphate group may be substituted with a sulfur, amine, or other group. Also considered as modified nucleotides are nucleotides having various other structural modifications not found ***in vivo*** without human intervention.

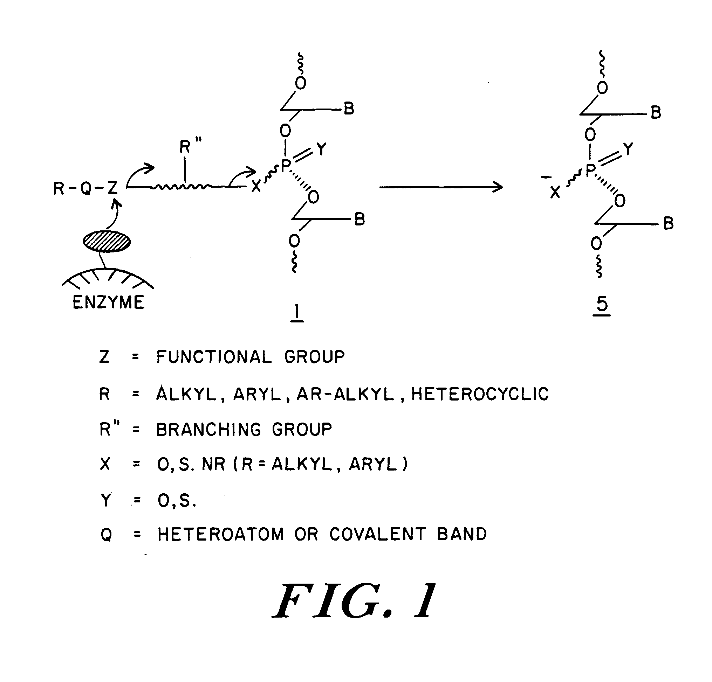

At least one nucleotide of the oligonucleotide prodrug has been derivatized such that the prodrug becomes less ionic and more lipophilic than it was before derivatization. This is accomplished by covalently attaching a lipophilic chemical group to the 3' phosphorothioate, 5' phosphorothioate, or internucleotidic phosphorothioate group of the nucleotide, shown as produg **1** in FIG. 1.

At least one nucleotide of the prodrug is derivatized as described above, and all of the nucleotides may be likewise derivatized. The derivatized nucleotides may be located anywhere in the oligonucleotide prodrug, i.e., they may be in the internal or terminal regions of the prodrug, or may be scattered throughout the molecule.

TABLE 1 below lists some representative oligonucleotide prodrugs having 6, 17, 25, and 30 nucleotides. "↓" indicates the position of the derivatized nucleotide residue.

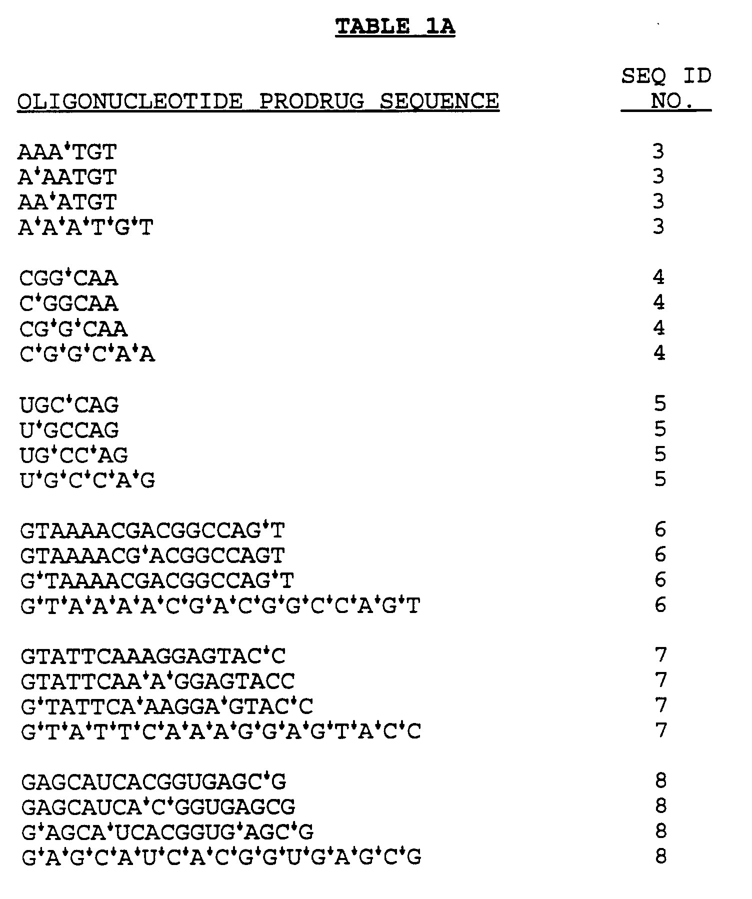

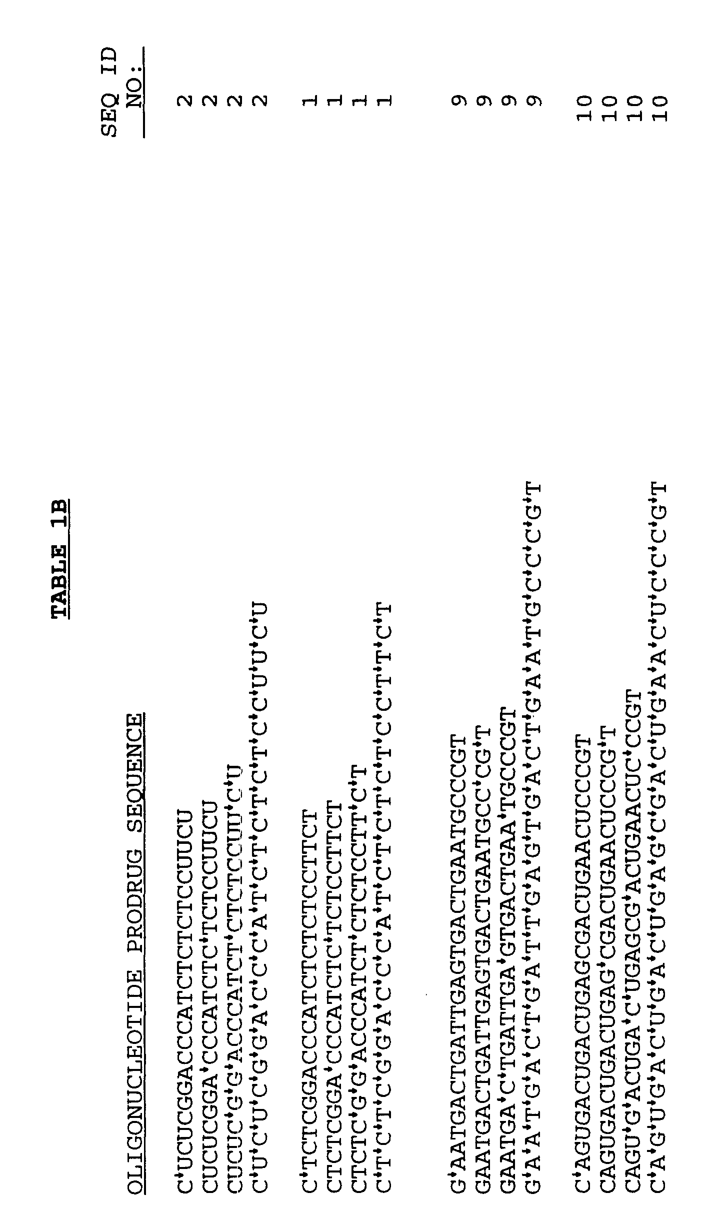

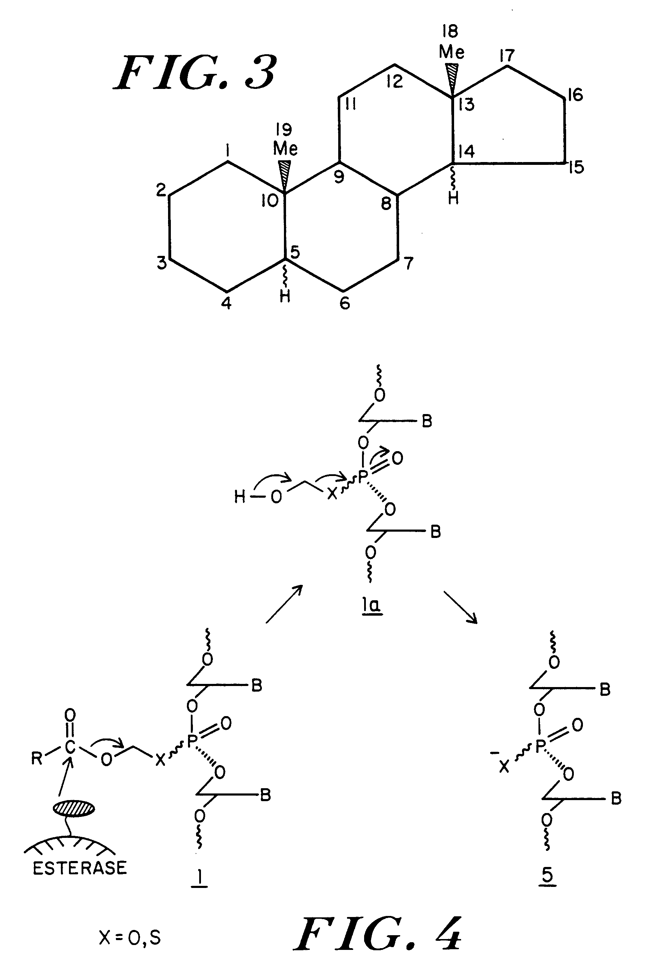

The derivatizing chemical group is lipophilic and decreases the ionic strength of the oligonucleotide as a whole. Useful lipophilic chemical groups are isobutyryloxymethyl or propanoyloxymethyl groups. FIG. 3 shows the general structure of a steroid having 4 carbon rings and 17 positions at which the lipophilic group may be attached. The lipophilic chemical groups attached to multiple derivatized nucleotides may be the same or different.

The sequence of the nucleotides in the oligonucleotide prodrugs of the invention may be any sequence, as the ability of the oligonucleotide prodrug to pass or be transported through cell membranes is not sequence-dependent. Thus, the sequence of nucleotides in the oligonucleotide prodrugs may vary according to the purpose for which the antisense oligonucleotide is being used. For example, if the oligonucleotide is being used to prevent or treat a specific viral infection, at least a portion of the nucleotide sequence of the prodrug will be complementary to a portion of the nucleotide sequence of the viral nucleic acid. Alternatively, the antisense oligonucleotide may be used to control the expression of a particular gene encoding a protein of interest in the target cell or tissue, such as an enzyme. The nucleotide sequences of many viruses and cellular genes are known and antisense oligonucleotides have been prepared thereto.

The oligonucleotide prodrugs of the invention are prepared by synthesizing the antisense oligonucleotide using nucleotides capable of derivatization, and then derivitizing or covalently linking the lipophilic chemical group to a reactive group on the oligonucleotide.

The parent antisense oligonucleotide of the invention can be prepared by any art recognized method (reviewed in ***Protocols For Oligonucleotides and Analogs* (*Meth. Mol. Bio.*** (Agrawal, ed.) Humana Press, Totowa, NJ, Volume 20, 1993); Goodchild (1990) ***Bioconjugate Chem.* 1**:165-187; and Uhlmann et al. (1990) ***Chem. Rev.* 90**:543-584). For example, nucleotides can be covalently linked using techniques such as phosphoramidate, H-phosphonate chemistry, methylphosphoramidate, or methoxyphosphoramidite chemistry which can be carried out manually or by an automated synthesizer and then processed.

The oligonucleotides of the invention may also be modified in a number of ways without compromising their ability to be derivatized or to hybridize to a target nucleic acid. For example, modifications include those which are internal or are at the end(s) of the oligonucleotide molecule and include additions to the molecule of the internucleoside phosphate linkages, such as cholesteryl or diamine compounds with varying numbers of carbon residues between the amino groups and terminal ribose, deoxyribose and phosphate modifications which cleave, or crosslink to the opposite chains or to associated enzymes or other proteins which bind to the viral genome. Examples of such modified oligonucleotides include oligonucleotides with a modified base and/or sugar such as arabinose instead of ribose, or a 3', 5'-substituted oligonucleotide having a sugar which, at both its 3' and 5' positions is attached to a chemical group other than a hydroxyl group (at its 3' position) and other than a phosphate group (at its 5' position). Other modified oligonucleotides are capped with a nuclease resistance-conferring bulky substituent at their 3' and/or 5' end(s) or have a substitution in one nonbridging oxygen per nucleotide. Such modifications can be at some or all of the internucleoside linkages, as well as at either or both ends of the oligonucleotide and/or in the interior of the molecule (reviewed in Agrawal et al. (1992) ***Trends Biotechnol.* 10**:152-158) .

The nucleotide in the oligonucleotide prodrug is derivatized with a lipophilic chemical group attached to the 3' phosphorothioate, 5' phosphorothioate, or internucleotide phosphorothioate group of at least one nucleotide in the oligonucleotide. Covalent linkage of the chemical group can be accomplished by any art recognized protocol specific for the group to be appended such as an amide or ester.

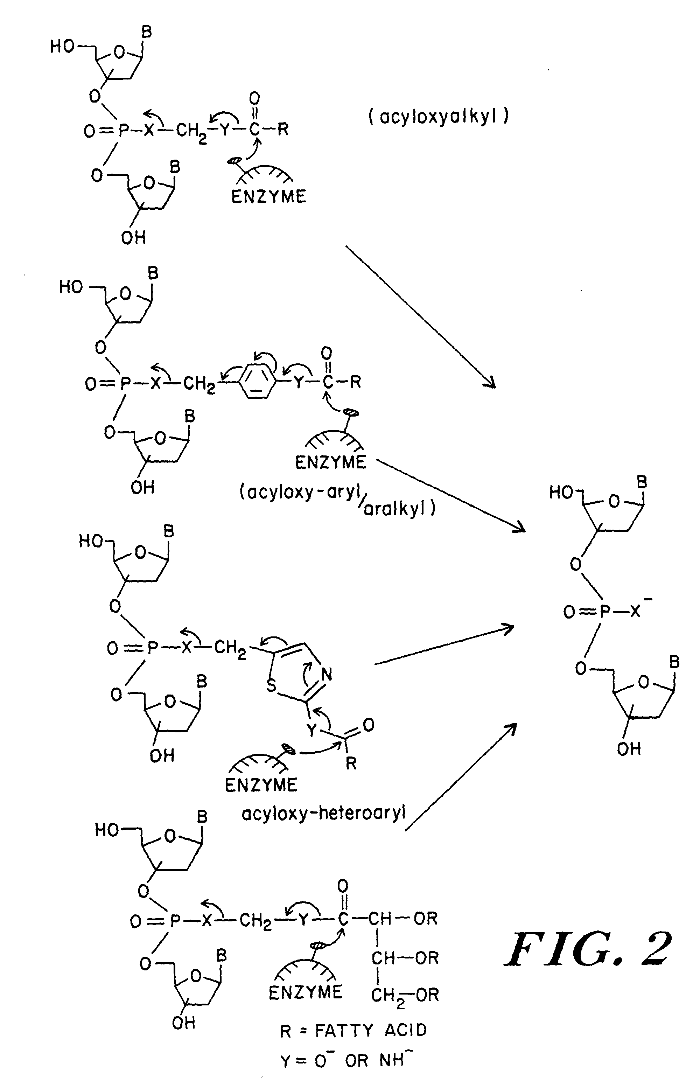

Once inside the cell, target tissue, or body in general, the oligonucleotide prodrug is processed by an endogenous enzyme such as esterase. This enzyme may be tissue- or cell-specific, and thus the oligonucleotide prodrug may be designed such that the lipophilic chemical groups are cleaved from the drug, thereby regenerating the parent antisense oligonucleotide only when the prodrug reaches or approaches the target tissue or cell. FIG. 1 depicts the generalized scheme of parent oligonucleotide (compound 5) regeneration from prodrug 1 with an enzyme, and FIG. 2 illustrates the specific action of an enzyme on various specific lipophilic chemical groups attached to the oligonucleotide prodrug.

Enzymes which release the lipophilic group from the oligonucleotide include esterases. Useful esterases found in cells and body tissues include but are not limited to thiol proteases, carboxyl proteases, metalloproteases, and serine proteases such as trypsin, chymotrypsin and elastase (found in the pancreas), thrombin, plasmin, and complement C1 (found in the serum), kallikrein (found in the blood and tissues), acrosomal protease (found in sperm), and lysosomal protease (found generally in animal cells).

For example, FIG. 4 shows the regeneration of an antisense oligonucleotide phosphorothioate, phosphorodithioate, or phosphoramidate from a prodrug form of the oligonucleotide (prodrug **1**) where X=S. In prodrug **1**, a labile carboxylic ester group has been incorporated so that an enzyme-mediated hydrolytic attack is directed to a highly electrophilic carbonyl carbon center rather than to the phosphoryl group of the phosphorothioate. This, in turn, ensures the regeneration of the phosphorothioate rather than the native phosphodiester backbone during the bio-activation (i.e., regeneration of the parent antisense oligonucleotide) ***in vivo.*** An acyloxyalkyl ester type group fulfills the requirements of an ideal appendage for the phosphorothioate oligonucleotides. Thus, the acyloxyalkyl ester type prodrug **1** undergoes bio-activation with an esterase to give the unstable hydroxymethyl oligonucleotide **1a** which then readily eliminates formaldehyde to give the parent phosphorothioate oligonucleotide **5**. The rate of enzymatic hydrolysis is modulated by choosing various acyl groups, with the more hindered derivatives such as prodrug **1** (where R = t-butyl) undergoing slower hydrolysis.

Incorporation of the acyloxyalkyl as well as aryl, alkyl, ar-alkyl, heterocyclic, fatty acid, steroid esters, and steroid amide groups into phosphorothioates, phosphorodithioates or phosphoramidates results in lipophilic and less ionic oligonucleotides. Such modifications enable these prodrugs to be efficiently taken up by cells, where cellular esterases or phosphoramidases hydrolyze the ester or amide group in the prodrug to regenerate the parent oligonucleotide.

The following methodology was designed for the chemoselective S-functionalization of an dinucleotide phosphorothioate as a model for the preparation of various oligonucleotide prodrugs. The dinucleoside phosphorothioate 2 [d(TpsT)] bearing the 5' dimethoxytrityl group (DMT) at the 5' end was synthesized on a 10 x 10 *µ* mole scale using known phosphoramidite chemistry on an automated DNA synthesizer (see, e.g., Beaucage et al. (1992) ***Tetrahedron* 48:**2223-2311). Oxidative sulfurization of the internucleotidic phosphite linkage was carried out using 3H-1,2-benzodithiole-3-one-1,1-dioxide to generate the phosphorothioate linkage as described by Iyer et al. *(****J****.* ***Org. Chem.*** (1990) **55**:4693-4698 and ***J. Am. Chem Soc.*** (1990) **112:**1253-1254). Following the synthesis, the controlled-pore-glass (CPG) support was treated with 28-30% NH₃ to cleave the dinucleoside phosphorothioate from the support and remove the β-cyanoethyl phosphate protecting group. The dimer was then subjected to reverse-phase HPLC to isolate the constituent Rₚ (retention time (Rₜ) = 39 min.) and Sₚ (Rₜ, 35 min.) diastereomers of phosphorothioate prodrug **2** bearing the 5'-DMT group. Each of the individual Rₚ and Sₚ diastereomers were then treated with 80% acetic acid to remove the 5'-DMT group and purified again by reverse phase HPLC to obtain pure Rₚ and Sₚ isomers (Rₚ:Rₜ, 24.2 min.; Sₚ:Rₜ 25.4 min.).

The assignment of absolute configurations "Rₚ" and "Sₚ" to the individual diastereomers of prodrug **2** follows the well-established literature precedent (Connolly et al. (1984) ***Biochem.* 23**:3443-3453), and is based on the relative mobilities of the Rₚ and Sₚ dinucleoside phosphorothioates (5'-DMT "on" and 5'-DMT "off") on reverse-phase HPLC.

To further confirm these assignments, the individual diastereomers of PS-prodrug **2** were treated with snake venom phosphodiesterase (type II) using the method of Connolly et al. (***ibid***.). The snake venom stereospecifically hydrolyzed the Rₚ diastereomer (Rₜ, 24.2 min., δ 52.6 ppm) and nuclease P1 which hydrolyzed the Sₚ diastereomer (Rₜ, 25.4 min., δ 52.2 ppm).

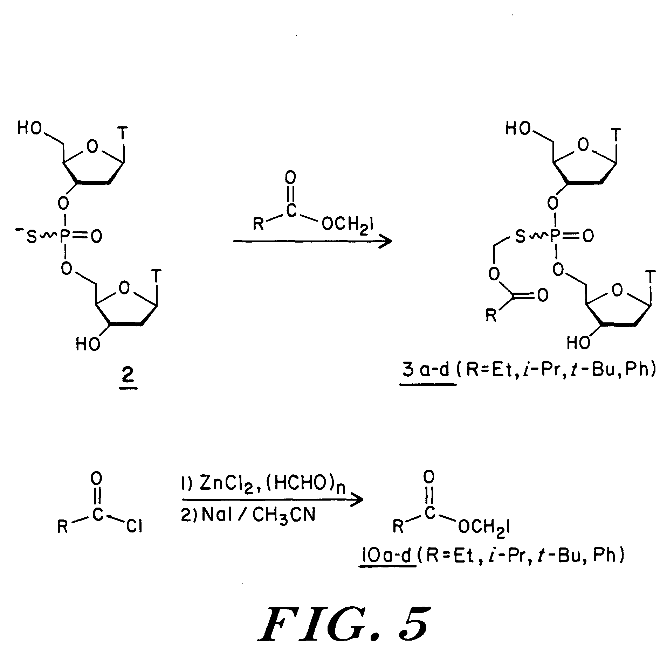

The diastereomers of prodrug **2** were converted to the S-alkyl phosphorothioates (PS-prodrugs **3a-d**) using a chemoselective S-alkylation protocol (e.g., Agrawal et al. (1991) ***Nucl. Acids Res*. 18**:5419-5423), as shown in FIG. 5. The iodoalkylacylates (compounds **10a-d**) required for the alkylation reactions were prepared from the corresponding chloroalkylacylates (FIG. 4) using the chloroalkylacylates according to the method of Iyer et al. **(*Tetrahedron Lett.*** (1989) **30**:7141-7144). These, in turn, were synthesized by the reaction of the corresponding acid chlorides with paraformaldehyde in presence of catalytic amounts of anhydrous zinc chloride, as described by Ulich et al. (***J****.* ***Am. Chem. Soc.*** (1921) **43:**660). The reactions were monitored by reverse-phase HPLC, and no evidence of any epimerization at the chiral phosphorous center was noted. Significant side products were not detected reflecting the lack of reactions at other sites. Thus, Rₚ2 gives Rₚ **3a-d**, and Sₚ **2** gives Sₚ **3a-d.**

In all cases, the reaction mixture was worked up and products isolated by preparative reverse-phase HPLC. The retention times of the various analogs of prodrug 3 are shown in TABLE 2.

**TABLE 2**

| Buffer Hydrolysis of **3a-c** | | | |
|---|---|---|---|
| Compound | Rₜ (min) | t_{1/2}(days) | % parent (analog **2)** |
| Rₚ **3a** | 38.7 | >5 | 22 |
| Sₚ **3a** | 39.3 | >5 | 20 |
| | | | |
| Rₚ **3b** | 41.6 | >10 | 25 |
| Sₚ **3b** | 42.2 | >10 | 25 |
| | | | |
| Rₚ **3c** | 44.3 | >30 | 46 |
| Sₚ **3c** | 44.9 | >30 | 35 |

As measured by ³¹P-NMR, the Rₚ isomer of analog **3c** typically had a value of 24.8 ppm and the Sₚ isomer of **3c** had a δ value 25.8 ppm. The Rₚ and Sₚ triesters of **3**, unlike the Rₚ and Sₚ diester counterparts (i.e., analog **2**) were resistant to hydrolysis by snake venom phosphodiesterase and P1 nuclease, respectively. These results indicate that the prodrug is less susceptible to nuclease digestion than is its parent.

Although prodrugs **3a-d** are phosphotriesters bearing a labile carboxylic ester moiety, they are easily isolated and purified; they are soluble in aqueous buffers and in organic solvents such as acetonitrile and chloroform. They can be stored indefinitely in aqueous buffers (pH 7.0) at 0-5°C with no evidence of decomposition. However, upon prolonged storage in aqueous buffers (pH 7.0) at ambient temperature, some decomposition occurs. TABLE 2 above shows the half-lives of decomposition of the analogs in aqueous buffers at ambient temperature. As would be expected, the less hindered analogs **3a** were more susceptible to hydrolytic decomposition than the more hindered analogs **3b-c**. The major product of decomposition was the desulfurized product, the natural diester, **4**.

Prodrug analogs **3a-3c** were then analyzed for their ability to undergo hydrolysis in serum. These serum-mediated hydrolysis studies were carried out on HPLC-purified materials containing ammonium acetate (i.e., salt). To determine whether the presence of salt had an impact on the kinetics and product profile of hydrolysis, (i.e., on bio-reversibility), esters **3a-c** (HPLC mobile phase containing or not containing salt were incubated with human serum. TABLE 3 shows the half-lives (t_{1/2}) of hydrolysis of analogs **3a-c** in the presence and absence of salt.

**TABLE 3**

| Hydrolysis of Analogs **3a-c** by Human Serum | | | | |
|---|---|---|---|---|
| Compound | Rₜ (min) | t_{1/2} (min) | k (x 10⁻²min⁻¹) | % compound 2 |
| *R*ₚ**3a** | 38.7 | 40 | 1.73 | 99 (*R*ₚ) |
| *S*ₚ**3a** | 39.3 | 11 | 6.30 | 99 (*S*ₚ) |
| | | | | |
| *R*ₚ**3b** | 41.6 | 82 | 0.85 | 99 (*R*ₚ) |
| *S*ₚ**3b** | 42.2 | 28 | 2.47 | 99 (*S*ₚ) |
| | | | | |
| *R*ₚ**3c** | 44.3 | 1980 | 0.04 | 95 (*R*ₚ) |
| *S*ₚ**3c** | 44.9 | 335 | 0.21 | 95 (*S*ₚ) |
| | | | | |
| *R*ₚ**3a*** | 38.7 | 13 | n.d. | 98 (*R*ₚ) |
| *S*ₚ**3a*** | 39.3 | 4.0 | n.d. | 99 (*S*ₚ) |
| | | | | |
| *R*ₚ**3b*** | 41.6 | 23 | n.d. | 97 (*R*ₚ) |
| *S*ₚ**3b*** | 42.2 | 5.0 | n.d. | 99 (*S*ₚ) |
| | | | | |
| *R*ₚ**3c*** | 44.3 | 163 | n.d. | 75 (*R*ₚ) |
| *S*ₚ**3c*** | 44.9 | 68 | n.d. | 87 (*S*ₚ) |

| | | | | |
|---|---|---|---|---|
| @ estimated at t_{1/2} of hydrolysis ^{*} no salt n.d. not determined | | | | |

In all cases, stereospecific hydrolytic conversion of the Sₚ triesters **3a-c** to the Rₚ **2** was observed. The Sₚ esters **3a-c** were hydrolyzed much faster compared to the Rₚ esters **3a-c.** In addition, the formation of significant amounts of phosphoric diester **4** as a by-product was seen upon hydrolysis of **3c**.

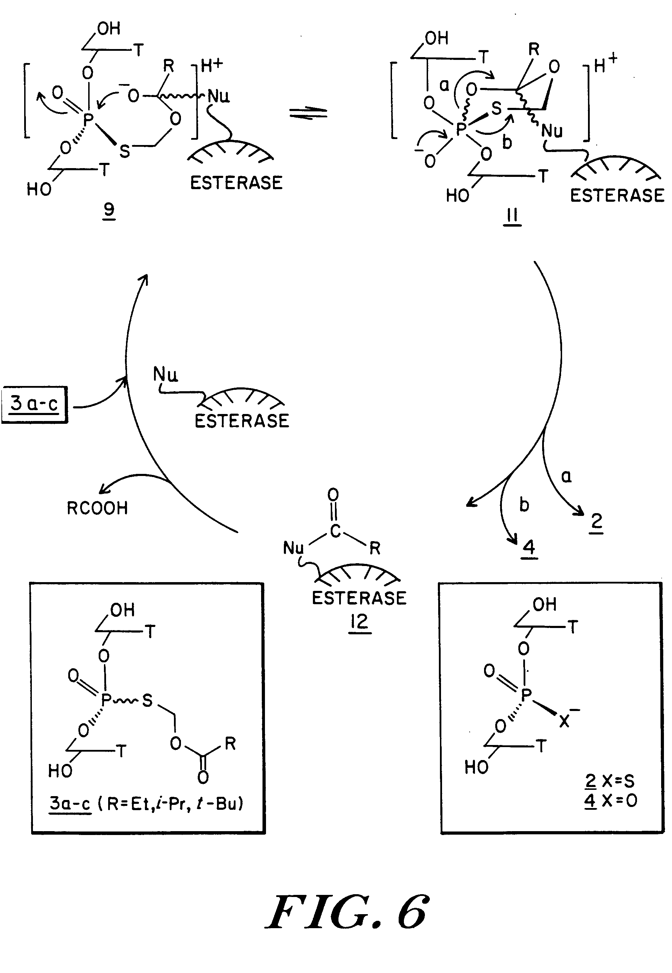

As TABLE 3 shows, when the same serum-mediated hydrolysis studies were done using salt-free materials, the half-lives of hydrolysis were significantly reduced. Typically, the half-life of hydrolysis Rₚ **3c**^{*} (salt free) was 163 minutes, whereas that of Rₚ **3c** (with salt) was 1,980 minutes. Increased formation of the desulfurized products **4** was also observed, especially in case of the hindered analogs Rₚ and Sₚ **3c,** when the enzyme-mediated hydrolysis was carried out in the absence of salt. In that event, the origin of the desulfurized product **4** in the case of analogs **3a**-c, is likely to follow the path shown in FIG. 6.

Alternatively, at least part of the desulfurized product may be formed due to hydrolysis mediated by a phosphodiesterase-like activity present in serum, and that ammonium acetate may suppress this phosphodiesterase-like activity and reduce esterase activity.

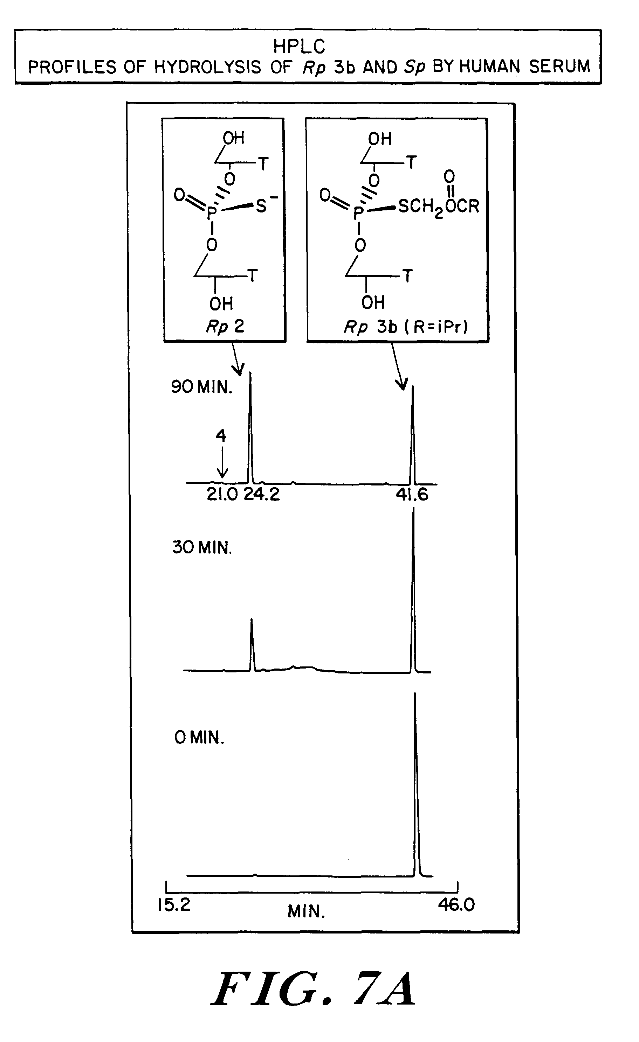

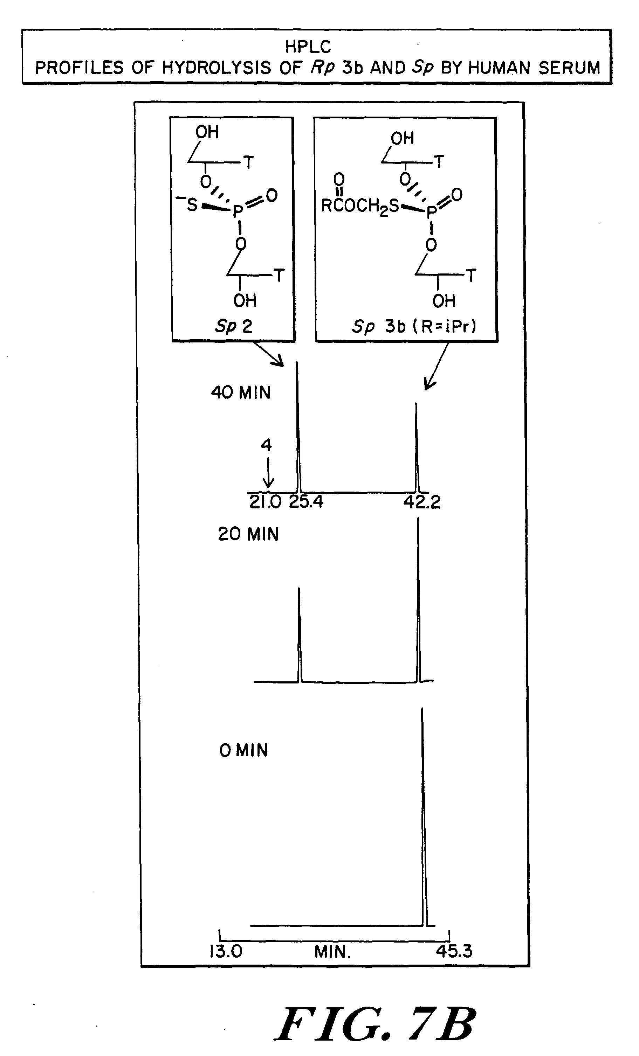

Studies were thus undertaken to confirm that the factor(s) present in serum which is responsible for mediating the hydrolysis of the esters has esterase-like activity. In these only salt-free materials were used. Porcine liver esterase (which is a mixture of at least seven enzymes) was used as a typical carboxyl esterase enzyme. The reactions were monitored by reverse-phase HPLC using a gradient of 100% ammonium acetate buffer (0.1 M) to 80% acetonitrile in ammonium acetate (0.1 M). The date obtained from these studies was analyzed according to a first order kinetic model. The results are shown in FIGS. 7A and 7B, where the arrows indicate the retention times in minutes of **4, 2**, and **3b**, and are summarized in TABLE 4.

**TABLE 4**

| Hydrolysis of 3a-c (Salt-Free) by Pig Liver Carboxy Esterase | | | |
|---|---|---|---|
| Compound | Rₜ(min) | t_{1/2}(min) | % compound 2^{@} |
| *R*ₚ**3a** | 38.7 | nd | n.d. |
| *S*ₚ**3b** | 39.3 | nd | n.d. |
| | | | |
| *R*ₚ**3b** | 41.6 | 42 | 99 (*R*ₚ) |
| *S*ₚ**3b** | 42.2 | 64 | 99 (*S*ₚ) |
| | | | |
| *R*ₚ**3c** | 44.3 | 185 | 97 (*R*ₚ) |
| *S*ₚ**3c** | 44.9 | 430 | 98 (*S*ₚ) |

| | | | |
|---|---|---|---|
| @ estimated at t_{1/2} of hydrolysis, remaining being **4**. n.d. not determined | | | |

Upon incubation of the substrates **3a-c** with pig liver esterase in a stoichiometric ratio (one unit/one *µ*mole of substrate), almost instantaneous stereospecific hydrolysis was observed to give the desired product Rₚ or Sₚ **2.** Under these conditions, no stereo-differentiation in the rates of hydrolysis of **3a-c** was noted (i.e., both Rₚ and Sₚ were hydrolyzed at the same rate). Also, no difference in the half-lives of the hindered and less hindered analogs was noted. These observations reflect a high binding affinity of the substrate for the enzyme and a fast catalyst rate. However, upon lowering the enzyme concentration, some stereo-differentiation was noted as previously observed in case of the serum studies. Inverse stereochemical preference was observed; Rₚ was hydrolyzed slightly faster (Rₚ **3c**, t_{1/2} = 185 min.) than Sₚ (Sₚ **3c**, t_{1/2} = 430 min.). These results suggest that pig liver esterases have different stereochemical specificities for substrates **3a-c** when compared to human plasma carboxyl esterase. As in case of the serum studies, the more hindered t-butyl analogs were hydrolyzed more slowly compared to the less hindered analogs. The formation of the desulfurized product **4** was also observed, especially in the case of hindered analogs **3c**, under these conditions as in case of the serum-mediated hydrolysis studies.

In order to get further insight into the mechanism of the hydrolysis reaction and to demonstrate that hydrolysis proceeds by initial attack on the carboxyl group, the Rₚ and Sₚ benzoyl analog **3d** was prepared. Incubation of Rₚ or Sₚ **3d** with pig liver esterase gave analog **2** along with the formation of benzoic acid which was identified by co-chromatographic comparison with an authentic standard. These data are indicative of an initial nucleophilic attack on the carbonyl carbon by esterases, rather than attack at the phosphoryl group, to generate analog **2**. A pathway for the formation of analog **4** is shown in FIG. 6. This pathway involves an initial nucleophilic attack by the serine hydroxyl group of the esterase on the ester carbonyl center to generate the oxy-anion intermediate **9** which performs an intramolecular attack on the juxta-positioned phosphorous center to give cyclic intermediate **11.** Fragmentation of intermediate **11** by ***path a*** gives the desired product **2,** whereas fragmentation of **11** by ***path b*** gives the desulfurized product **4,** each pathway generating the same acyl-enzyme intermediate **12.** The enzyme-mediated hydrolysis gives the expected phosphorothioate **2** (by ***path a)*** as the predominant product.

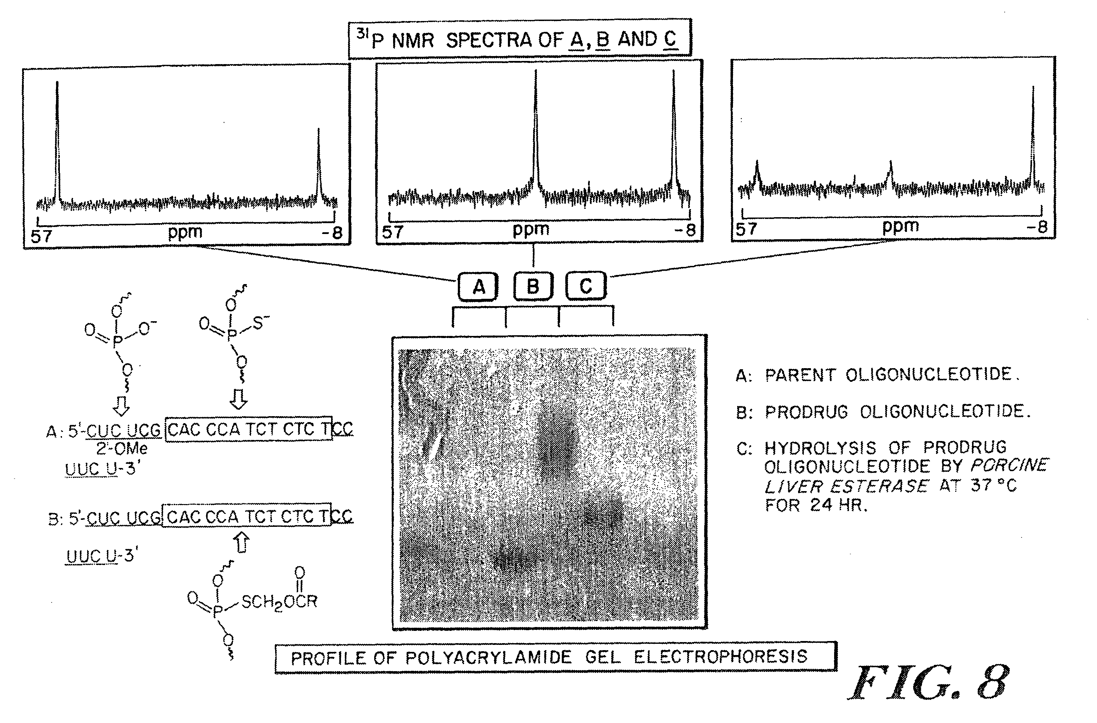

Based on the results of the model described above, an oligonucleotide prodrug and its parent oligonucleotide, both having SEQ ID NO:2, were examined before and after hydrolysis with an esterase by NMR spectroscopy and polyacrylamide gel electrophoresis. The oligonucleotides were dissolved in D₂O, and the NMR spectra recorded. The results are shown in FIG. 8 wherein A is the spectrum of the parent oligonucleotide, B is the spectrum of the prodrug, and C is the spectrum of the prodrug that had been incubated with an esterase.

As shown in FIG. 8, a chemical shift is seen in the spectrum of the phosphorus nuclei which have been derivatized with a lipophilic group (in A it is at about δ 57, wherein in B, it has shifted to the right). Furthermore, a shift in the spectrum of the derivatized phosphorus nuclei back to the position it was at in the parent after 24 hours of esterase digestion (δ-8) is seen in C, demonstrating the reversibility of the derivatization.

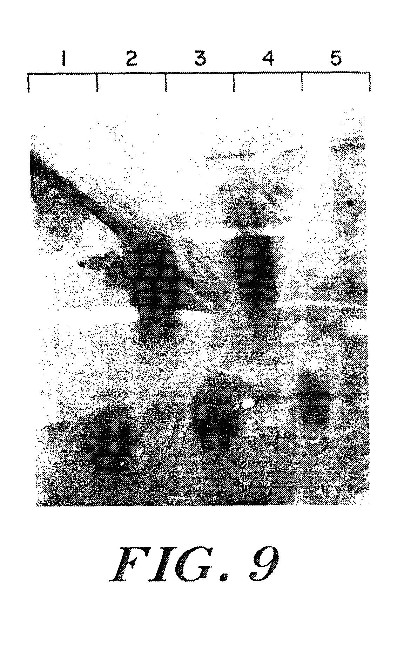

The species analyzed in A, B, and C were subjected to polyacrylamide gel electrophoresis and autoradiography, as shown in FIGS. 8 and 9. These autoradiograms demonstrate that the oligonucleotide prodrug is converted back to the parent oligonucleotide within 36 hours of incubation with the enzyme.

The following examples illustrate the preferred modes of making and practicing the present invention, but are not meant to limit the scope of the invention since alternative methods may be utilized to obtain similar results.

### EXAMPLES

### 1. Synthesis of d(TpsT) and Parent Oligonucleotides

The automated solid-phase synthesis of d(TpsT) 2 and oligonucleotides was carried out on a 10 x 10 *µ*mole scale on a DNA synthesizer (Biosearch 8700, Bedford, MA using phosphoramidite chemistry (Beaucage et al. (1992) ***Tetrahedron* 48**:2223-2311). The oxidative sulfurization reaction required for the preparation of oligodeoxyribonucleoside phosphorothioates was effected by a 0.2 M solution of crystalline. 3***H***-1,2-benzodithiole-3-one-1,1-dioxide (R.I. Chemical Co., Costa Mesa, CA), in acetonitrile as described by Iyer et al. ***(J. Am. Chem. Soc.*** (1990) **112**:1253-54; ***J. Org. Chem.*** (1990) **55**:4693-98). The sulfurization reaction was performed over a period of 45 seconds to 2 minutes depending on the scale of synthesis. Following synthesis, the controlled pore glass (CPG) support was treated with 28-30% NH₃ at 55°C for 8-10 hours to cleave the dinucleoside phosphorothioate from the support and remove the β-cyanoethyl phosphate protecting group. The Rₚ:Sₚ ratio of 2 was estimated to be 60:40 based on ³¹P-NMR and HPLC analysis.

### 2. Synthesis of Iodoalkyl Acylates

Iodoalkyl acylates **10a-d** were prepared and
characterized as previously described by Srivastva et al. **(*Bioorg. Chem.*** (1984) **12:**118-129), and by Iyer et al. **(*Tetrahedron. Lett. (1989)* 30:**7141-7144). Briefly, to a 117 mM solution of sodium iodide (17.56) in 100 ml dry acetonitrile, was added 12.70 g (85 mM) chloroalkyl acylate over a period of 30 minutes at 25°C in the dark. A white precipitate of NaCl began to appear immediately. The contents were stirred for 12 hours. The precipitate was filtered, and the acetonitrile was removed from the filtrate ***in vacuo.*** The filtrate was taken up in 70 ml toluene, washed two times with 40 ml 5% aqueous sodium bisulfite, and then 40 ml water. The toluene layer was then dried over anhydrous sodium sulfate. Toluene was removed ***in vacuo*** and distillation of the resulting pale yellow oil gave a clear, colorless liquid (48-50°C, 3 mm Hg, 14.2 g, 70%) ¹H-NMR (CDCl₃) δ ppm 1.19 (s, 9H), 5.91 (s, 2H) ¹³C-NMR (CDCl₃) : δ ppm 26.4 (CH₃), 3L4 (CH₂), 38.7 (-C), 176.0 (CO)). The distilled products **10a-d** were stored at -80°C until ready to use.

### 3. Synthesis of Dinucleoside S-alkyl Phosphorothiolates

The esters **3a-c** were synthesized by reacting 50 A₂₆₀ units of Rₚ or Sₚ **2** in (0.5 ml 250 mM Tris buffer, pH 7.0) with the corresponding iodoalkyl acylates **10a-d** (2 mmoles) in 3 ml acetonitrile, at 37°C for 3-4 hr. The reaction mixture was quenched with 100 *µ*l 0.5% sodium bisulfite, evaporated to dryness ***in vacuo*** and subjected to preparative reverse-phase HPLC as described below. The solvent was removed ***in vacuo*** and the esters **(3a-c**) thus obtained (isolated yields 60-70% based on compound 2), were used as such for further studies. NMR spectra were recorded on a spectrometer operating in the presence of broad band decoupling at 7.05 Tesla (300 MHz for 'H. ³¹P-NMR spectra were recorded in deuterated solvents using trimethylphosphate as the external reference. Typical ³¹P-NMR (D₂O) : δ Rₚ **3c**, 24.8; Sp **3c** 25.8 ppm.

### 4. Preparative HPLC

Deprotected TpsT dimer bearing the DMT group at the 5'-end was purified by reverse-phase HPLC using a C-18 reverse-phase column (125Å, 55-105 *µ*M, WATERS (Milford, MA), and a gradient of 100% A to 100% B over 70 minutes [A: CH₃CO₂NH₄ (0.1 M in water); B: acetonitrile: CH₃CO₂NH₄ (0.1 M) (80:20)], using a flow rate of 12 ml/min. The TpsT DMT-on peaks (Rₜ = 41 and 45 min) were collected and subjected to detritylation using 80% acetic acid for 30 min. The solvent was removed and the crude compound **2** subjected to reverse-phase HPLC as described below using a C-18 column developed with a gradient of 100% A to 100% B over 70 min, using either A, (0.1 M CH₃CO₂NH₄ in water) ; B (acetonitrile : 0.1 M CH₃CO₂NH₄, ^{*}80:20) or A (water) and B (acetonitrile : water (80:20)). Use of the latter system afforded salt-free materials. The Rₚ and Sₚ **2** fractions were collected, evaporated, lyophilized and stored at 0°C until ready to use. ³¹P-NMR (D₂O) : δ Rₚ 2, 52.6 and S_{P} **2**, δ 52.2 ppm.

### 5. Bioreversibility Studies

### A. Hydrolysis With Snake Venom Phosphodiesterase.

To confirm the Rₚ and Rₛ assignments, the individual diastereomers of **2** were treated with snake venom phosphodiesterase (type II) which stereospecifically hydrolyzed the Rₚ diastereomer (Rₜ = 24.2 min., δ = 52.6 ppm) and nuclease P1 which hydrolyzed the Sₚ diastereomer (Rₜ = 25.4 min., δ = 52.2 ppm). This was accomplished by using the method of Connolly et al. (***Biochem.*** (1984) **23**:3443-3453). Snake venom phosphodiesterase was obtained from Boehringer Mannheim GmbH, Indianapolis, Indiana, in a suspension of 50% glycerol, pH 6.0.

### B. Hydrolysis With Buffer

The hydrolysis mixture contained about. 0.6 A₂₆₀ units of substrates 3a-3c in 80 *µ*l 25 mM Tris buffer, pH 7.0 at 37°C. At each time point, 10 *µ*l aliquots of incubation mixture were diluted with 140 *µ*l buffer A and analyzed by reverse-phase HPLC, (600E instrument, Waters, Milford, MA) using a C18 4*µ* Radial Pak cartridge column (Waters, Milford, MA), developed with a gradient (100% A to 60% B over 60 minutes) of buffer A (0.1 M CH₃CO₂NH₄)) and buffer B (80:20, CH₃CN:0.1 M CH₃CO₂NH₄), with a flow rate 1.5 ml/min. Retention times (Rₜ) of Rₚ **2,** were 24.2; Sₚ **2,** 25.4; and **4,** 21.0 minutes respectively. Prodrugs **3a-3c** were converted back to the starting dinucleotides after exposure to buffer.

### C. Serum Hydrolysis

The hydrolysis mixture contained about 0.6 A₂₆₀ units of substrates **3a-3c**, 20 µl human serum (GIBCO, BRL, Gaithersburg, MD) in 60 µl of 25 mM Tris buffer, pH 7.0 at 37°C. At each time point, aliquots of incubation mixture were diluted with 140 *µ*l buffer A and analyzed by reverse-phase HPLC, as described in EXAMPLE 5B above. Prodrugs **3a-3c** were converted back to the starting dinucleotides after exposure to serum, as shown in FIG. 7A and 7B.

### D. Hydrolysis With Porcine Liver Esterase

The hydrolysis mixture contained about 0.6 A₂₆₀ units of substrates **3a-3c** and µl of pig liver carboxyl esterase in 60 µl of 25 mM Tris buffer, pH 7.0) at 37°C. At each time point, 10 µl aliquots of incubation mixture were diluted with 140 µl buffer A and analyzed by reverse-phase HPLC, as described in EXAMPLE 5B above. Prodrugs 3a-3c were converted back to the starting dinucleotides after exposure to the pig liver esterase.

### 6. Preparation of Oligonucleotide Prodrugs

To a solution of 90 O.D. of an oligonucleotide (SEQ ID NO:1 or SEQ ID NO:2) in 0.5 ml 250 mM Tris-HCl buffer, pH 7.0, was added 20 µl iodomethyl isobutyrate in 0.5 ml acetonitrile. The solution was incubated at 37°C for 1-3 hr. The pH of the solution was maintained around 6-7 by adding trace amounts of triethyl amine periodically. At the end of the reaction, the solvent was removed under pressure and the residue dissolved in 200-500 µl water and 30-40 µl 1 M sodium chloride solution. To the solution was added 1-1.2 ml cold ethanol, and the solution kept at about -80°C for 1-2 hr. The solution was centrifuged at 10,000 g for 15 minutes, and the resulting pellet analyzed by HPLC and gel elctrophoresis, or dissolved in sodium chloride solution and ethanol precipitated as above.

As shown in FIG. 8, ³¹P-NMR of this product showed a signal at δ 25 ppm as compared to the starting oligonucleotide at δ 51 ppm. Analysis by gel electrophoresis (20% polyacrylamide) of the product showed a slow moving band as compared to the starting oligonucleotide.

### 7. Bio-reversibility Studies with Oligonucleotide Prodrugs

### A. Hydrolysis with Pig Liver Esterase

To 1.5 A₂₆₀ units of prodrug (in 25 µl 250 mM Tris, pH 7.2) was added 2 *µ*l pig liver esterase and the reaction mixture incubated at 37°C overnight. Aliquots of the reaction mixture were then analyzed by gel electrophoresis using a 20% polyacrylamide, 7 M urea denaturing gel. FIGS. 8 and 9 show the profile of the reaction mixture obtained after 24 and 36 hours, respectively. The prodrug oligonucleotide is converted back to the parent oligonucleotide after exposure to the pig liver esterase.

### B. Hydrolysis with Serum

To 1.5 A₂₆₀ units of the prodrug (25 *µ* l) oligonucleotide in 250 mM Tris buffer, pH 7.2) was added 40 *µ*l human serum, and the reaction mixture incubated at 37°C overnight. Aliquots of the reaction mixture was analyzed by gel electrophoresis as described in EXAMPLE 7A. FIG. 8 shows the profile obtained of the incubate after 24 hours. The prodrug oligonucleotide is converted back to the starting oligonucleotide after exposure to serum.

### C. In vivo Hydrolysis

Hybrid, chimeric prodrugs having SEQ ID NO:2 and a combination of 2-O-methyl ribonucleotides and phosphorothioates were administered in normal saline as a bolus intravenous injection into the tail vein of 150-200 g Sprague Dawley or albino rats. Three rats were used for each dose to provide doses of 1-10 mg/Kg. After administration, the animals were placed in metabolism cages and urine samples were collected for up to 72 hours. 0.25 ml blood samples are collected from the cut axilla region at period intervals following dosing. The samples were collected in microfuge tubes containing 0.25 *µ* l of 27.5 mM EDTA at 0°C and centrifuged at 16,000 x g speed.

The plasma samples (150-200 *µ*l) were analyzed by polyacrylamide gel electrophoresis (PAGE) in a 20% polyacrylamide, 7 M urea denaturing gel. These samples were also analyzed by HPLC to determine the half-life of bio-reversibility of the oligonucleotide prodrugs to parent oligonucleotide. The urine samples are also analyzed by PAGE and HPLC to determine content of the oligonucleotide prodrug and its metabolites. ³⁵S-labelled oligo-prodrugs are used in these studies.

Anti-HIV screening of the oligonucleotide prodrugs in chronically HIV-infected cells is conducted as described in Lisciewicz et al. (1993) ***Proc. Natl. Acad. Sci. (USA)* 90:**3860-3864.

These studies illustrate that the above-described specifically embodied oligonucleotide prodrugs of the invention, in addition to having favorable physicochemical and pharmacologic properties, have good therapeutic potential against AIDS.

### 8. Dinucleoside S-acyloxyaryl Phosphorothioate Prodrugs

In these prodrugs, generally depicted as indicated in Figure 2, the acyloxyaryl group is linked to the phosphorothioate group via a methylene bridge. Incorporation of the aryl group in the appendage confers a certain degree of conformational rigidity to the appendage, providing greater stability of the prodrug in aqueous buffers within a wide pH range.

Acyloxyaryl phosphorothioate prodrugs were synthesized by reaching the underivatized phosphorothioate with 4-O-isobutyryl-α-iodotoluene, which was synthesized as described below.

Commercially available 4-hydroxybenzyl alcohol (Aldrich, Milwaukee, WI) was evaporated three times from pyridine, then dissolved in pyridine to yield 0.2 M 4-hydroxybenzyl alcohol. Chlorotrimethylsilan (Aldrich) was added in 1.2 molar equivalents, and the solution was stirred for fifteen minutes at room temperature. Isobutyryl chloride (Aldrich) was added in 1.2 molar equivalents, and the reaction was stirred for two hours. The reaction mixture was cooled to 0°C in an ice bath and excess water was added (50 equivalents). The ice bath was removed, and the reaction was stirred for four hours. The reaction mixture was concentrated and extracted with ethyl acetate. The organic layer was washed with 10% sodium bicarbonate solution. Evaporation yielded an oily residue which was purified by column chromatography on silica gel using hexane:ethyl acetate (80:20) as the eluent. Evaporation gave 4-O-isobutyryl benzyl alcohol as a colorless oil in 80-90% yield.

The 4-O-isobutyryl benzyl alcohol was then dissolved in a 1:2 mixture of ether:acetonitrile to a concentration of 0.28 M. Triphenyl phosphine, imidazole, and sublimed iodine were added in 1.5 molar equivalents each, and the reaction was stirred for two hours. The reaction mixture was extracted with ether. Concentration of the ether layer was followed by chromatography on a silica column, using hexane:ethyl acetate (90:10) as eluent. The fractions containing the product were concentrated to dryness and 4-O-isobutyryl-α-iodotoluene was obtained as a white solid in 90-95% yield.

4-O-isobutyryl-α-iodotoluene (27 mg) was dissolved in a 50:50 mixture of pH 7 Tris HCl buffer (0.5 M):acetonitrile. This solution was added to 30 O.D. units of TpsT and kept at 37°C for three hours. Every half hour, the pH was adjusted with triethylamine (Aldrich) to maintain pH = 6-7. After three hours, the reaction was complete as evaluated by HPLC.

The acyloxybenzyl dinucleoside phosphorothioate was obtained as an Rₚ, Sₚ mixture, which is a substrate for Porcine liver esterases (Sigma). Incubation of the acyloxybenzyl dinucleoside phosphorothioate with esterases resulted in rapid, stereospecific, and quantitative conversion to the parent phosphorothioate, with a quinomethide as a byproduct of the hydrolysis. The t_{½} of the Rₚ acyloxybenzyl dinucleoside phosphorothioate was eight hours, and that of the Sₚ stereoisomer was twelve hours. In addition, no desulfurized product resulted from hydrolysis of the prodrugs.

The half-lives of degradation of the prodrugs in buffers ranging from pH 2 to pH 8 was greater than 30 days at 22°C.

## Claims

1. An oligonucleotide prodrug comprising at least six covalently linked nucleotides, at least one nucleotide being derivatized with a lipophilic chemical group reversibly and covalently attached to the nucleotide at a 5' phosphorothioate, a 3' phosphorothioate, or an internucleotidic phosphorothioate linkage;
the lipophilic chemical group being an isobutyryloxymethyl group or a propanoyloxymethyl group; and
the prodrug being reactive with a cellular esterase which cleaves the lipophilic group from the derivatized nucleotide.

2. The oligonucleotide prodrug of claim 1 which is chimeric.

3. The oligonucleotide prodrug of claim 1 comprising at least one deoxyribonudeotide and at least one ribonucleotide.

4. The oligonucleotide prodrug of claim 3 wherein the ribonudeotide is a 2'-O-alkyl ribonucleotide.

5. A pharmaceutical formulation comprising the oligonudeotide prodrug of claim 1.

6. A pharmaceutical formulation of claim 5 wherein the oligonudeotide prodrug comprises a nucleic acid sequence complementary to a region of a viral nucleic acid.

7. The pharmaceutical formulation of claim 6, further comprising a second antiviral agent.

8. The pharmaceutical formulation of claim 7, wherein the oligonudeotide prodrug comprises a nucleic acid sequence complementary to a first region of the viral nucleic acid, and the second antiviral agent is a second oligonudeotide having a nucleotide sequence complementary to a second region of the viral nucleic acid which does not overlap with the first region.

9. The pharmaceutical composition of claim 5 in an orally tolerable carrier.

10. The use of the oligonudeotide prodrug of claim 1 in vitro to increase intracellular concentration of an exogenous oligonucleotide.

11. The use of the oligonucleotide prodrug of claim 1 for the preparation of a pharmaceutical composition to increase intracellular concentration of an exogenous oligonucleotide.

12. The use of the oligonucleotide prodrug of claim 1 in vitro to treat or to prevent viral infection in a cell.

13. The use of the oligonudeotide prodrug of claim 1 for the preparation of a pharmaceutical composition to treat or prevent viral infection in a cell.

## Patentansprüche

1. Oligonukleotid-Arzneistoff-Vorläufer, der mindestens sechs kovalent verbundene Nukleotide umfasst, wobei mindestens ein Nukleotid mit einer lipohilen chemischen Gruppe derivatisiert ist, die reversibel und kovalent an das Nukleotid an ein 5'-Phosphorothioat, ein 3'-Phosphorothioat oder eine Internukleotid-Phosphorothioat-Bindung gebunden ist, wobei
die lipophile chemische Gruppe eine Isobutyryloxymethylgruppe oder eine Propanoyloxymethylgruppe ist und
der Arzneistoff-Vorläufer mit einer zellulären Esterase reaktiv ist, die die lipophile Gruppe von dem derivatisierten Nukleotid abspaltet.

2. Oligonukleotid-Arzneistoff-Vorläufer gemäß Anspruch 1, der ein Chimär ist.

3. Oligonukleotid-Arzneistoff-Vorläufer gemäß Anspruch 1, der mindestens ein Desoxyribonukleotid und mindestens ein Ribonukleotid umfasst.

4. Oligonukleotid-Arzneistoff-Vorläufer gemäß Anspruch 3, wobei das Ribonukleotid ein 2'-O-Alkyl-Ribonukleotid ist.

5. Pharmazeutische Formulierung, die den Oligonukleotid-Arzneistoff-Vorläufer gemäß Anspruch 1 umfasst.

6. Pharmazeutische Formulierung gemäß Anspruch 5, wobei der Oligonukleotid-Arzneistoff-Vorläufer eine Nukleinsäuresequenz umfasst, die zu einer Region einer viralen Nukleinsäure komplementär ist.

7. Pharmazeutische Formulierung gemäß Anspruch 6, die ferner einen zweiten antiviralen Wirkstoff umfasst.

8. Pharmazeutische Formulierung gemäß Anspruch 7, wobei der Oligonukleotid-Arzneistoff-Vorläufer eine Nukleinsäuresequenz umfasst, die zu einer ersten Region der viralen Nukleinsäure komplementär ist, und der zweite antivirale Wirkstoff ein zweites Oligonukleotid mit einer Nukleotidsequenz ist, die zu einer zweiten Region der viralen Nukleinsäure komplementär ist, die nicht mit der ersten Region überlappt.

9. Pharmazeutische Zusammensetzung gemäß Anspruch 5 in einem oral verträglichen Träger.

10. Verwendung des Oligonukleotid-Arzneistoff-Vorläufers gemäß Anspruch 1 *in vitro* für eine Erhöhung der intrazellulären Konzentration eines exogenen Oligonukleotids.

11. Verwendung des Oligonukleotid-Arzneistoff-Vorläufers gemäß Anspruch 1 für die Herstellung einer pharmazeutischen Zusammensetzung für die Erhöhung der intrazellulären Konzentration eines exogenen Oligonukleotids.

12. Verwendung des Oligonukleotid-Arzneistoff-Vorläufers gemäß Anspruch 1 *in vitro* für die Behandlung oder Vermeidung einer viralen Infektion in einer Zelle.

13. Verwendung des Oligonukleotid-Arzneistoff-Vorläufers gemäß Anspruch 1 für die Herstellung einer pharmazeutischen Zusammensetzung für die Behandlung oder Vermeidung einer viralen Infektion in einer Zelle.

## Revendications

1. Promédicament oligonucléotidique comprenant au moins six nucléotides liés par covalence, au moins un nucléotide étant dérivatisé avec un groupe chimique lipophile fixé de manière réversible et par covalence au nucléotide sur un 5' phosphorothioate, un 3' phosphorothioate, ou une liaison phosphorothioate intemucléotidique;
le groupe chimique lipophile étant un groupe isobutyryloxyméthyle ou un groupe propanoyloxyméthyle; et
le promédicament étant réactif avec une estérase cellulaire qui clive le groupe lipophile du nucléotide dérivatisé.

2. Promédicament oligonucléotidique selon la revendication 1, qui est chimérique.

3. Promédicament oligonucléotidique selon la revendication 1, comprenant au moins un désoxyribonucléotide et au moins un ribonucléotide.

4. Promédicament oligonucléotidique selon la revendication 3, dans lequel le ribonucléotide est un 2'-O-alkyl ribonucléotide.

5. Formulation pharmaceutique comprenant le promédicament oligonucléotidique selon la revendication 1.

6. Formulation pharmaceutique selon la revendication 5, dans laquelle le promédicament oligonucléotidique comprend une séquence d'acide nucléique complémentaire d'une région d'un acide nucléique viral.

7. Formulation pharmaceutique selon la revendication 6, comprenant en outre un second agent antiviral.

8. Formulation pharmaceutique selon la revendication 7, dans laquelle le promédicament oligonucléotidique comprend une séquence d'acide nucléique complémentaire d'une première région de l'acide nucléique viral, et le second agent antiviral est un second oligonucléotide ayant une séquence nucléotidique complémentaire d'une seconde région de l'acide nucléique viral, qui ne se chevauche pas avec la première région.

9. Composition pharmaceutique selon la revendication 5 dans un support tolérable par voie orale.

10. Utilisation du promédicament oligonucléotidique selon la revendication 1 in vitro pour augmenter la concentration intra-cellulaire d'un oligonucléotide exogène.

11. Utilisation du promédicament oligonucléotidique selon la revendication 1 pour la préparation d'une composition pharmaceutique pour augmenter la concentration intra-cellulaire d'un oligonucléotide exogène.

12. Utilisation du promédicament oligonucléotidique selon la revendication 1 in vitro pour traiter ou pour prévenir une infection virale dans une cellule.

13. Utilisation du promédicament oligonucléotidique selon la revendication 1 pour la préparation d'une composition pharmaceutique pour traiter ou prévenir une infecton virale dans une cellule.
